# EUROPEAN PATENT SPECIFICATION

(11) **EP 3 020 420 B1**
(45) Date of publication and mention of the grant of the patent: **04.09.2019**
(21) Application number: 15193395.9
(22) Date of filing: 06.11.2015
(51) Int. Cl.: A61L 2/07, B01D 15/12, C02F 1/00, C02F 9/00, C02F 1/02, C02F 1/28, C02F 1/42, C02F 103/02

(54) **STERILISING APPARATUS AND USE OF SAID APPARATUS COMPRISING AUTOCLAVE AND EXTERNAL WATER PURIFICATION FILTER**
STERILISIERUNGSGERÄT UND VERWENDUNG DES GERÄTS UMFASSEND EINEN AUTOKLAV UND EINEN EXTERNEN WASSERFILTER
APPAREIL DE STÉRILISATION ET UTILISATION D'UN TEL APPAREIL COMPRENANT UN AUTOCLAVE ET UN FILTRE D'EAU EXTERNE

(30) Priority: 14.11.2014 IT MI20141975
(43) Date of publication of application: 18.05.2016
(73) Proprietor: Absolute Up S.r.l., 24020 Villa di Serio (BG) (IT)
(72) Inventor: ONGARO, Daniele, I - 24020 VILLA DI SERIO (Bergamo) (IT); GHILARDI, Maria Pia, I - 24020 VILLA DI SERIO (Bergamo) (IT)
(74) Representative: Lunati & Mazzoni S.r.L.

(56) References cited:
- WO-A1-02/096472
- WO-A1-2006/003353
- WO-A1-2014/141063
- WO-A2-2013/093700
- AU-B3- 649 600
- CN-A- 102 961 772
- CN-U- 203 252 938
- JP-A- H05 185 069
- US-A- 5 880 438
- US-A1- 2006 163 137

## Description

The present invention relates to a steriliser or steam disinfector hereinafter autoclave in particular for medical-dental use of the type specified in the preamble to the first claim.

Similar devices are described by patent applications US-A-5880438, WO-A-2013/093700, WO-A-02/096472, WO-A-2014/141063.

Autoclaves of the type for medical-dental use are currently known of.

They are supplied with demineralised and purified water which is brought by said autoclave to high temperatures and pressures. The water in a steam state is then channelled into an insulated chamber in which the various items to be sterilised, in particular medical or medical-dental instruments, are placed.

The steam performs a sterilisation cycle, at specific temperatures and pressures, even variable, for a given amount of time after which it is expelled into the environment, possibly after cooling.

The supply of demineralised and purified water usually takes place through access means, such as hatches or the like, directly inside the tank, in turn inside the autoclave.

Alternatively, some manufacturers propose a supply consisting of an outer tank, easy to fill and connected to the inner tank by means of pipes and the like.

The prior art described above entails several significant drawbacks.

In particular the supply of demineralised and purified water is expensive and laborious for the user of the autoclave.

In addition, the handling and possible dispersion of the condensed water into the environment after the sterilisation cycle is harmful to the environment.

Yet another drawback is the fact that the production of demineralised and purified water has a high energy cost.

In this situation the technical purpose of the present invention is to devise a sterilisation method using an autoclave able to substantially overcome the drawbacks mentioned above.

Within the sphere of said technical purpose one important aim of the invention is to devise a sterilisation method using an autoclave which permits a simple and economic supply of water for the sterilisation.

A further technical task of the present invention is to obtain a sterilisation method using a simple autoclave the maintenance of which is simple and safe.

A further, no less important aim of the present invention is to obtain a sterilisation method using an autoclave for sterilisation which permits energy saving in the production of demineralised and purified water.

The technical purpose and specified aims are achieved by a sterilisation method using an autoclave as claimed in the appended Claim 1.

Preferred embodiments are evident from the dependent claims.

The characteristics and advantages of the invention are clearly evident from the following detailed description of a preferred embodiment thereof, with reference to the accompanying drawings, in which:
**Fig. 1** shows a schematic view of the autoclave for sterilisation according to the invention;
**Fig. 2** shows a portion of the autoclave for sterilisation according to the invention; and

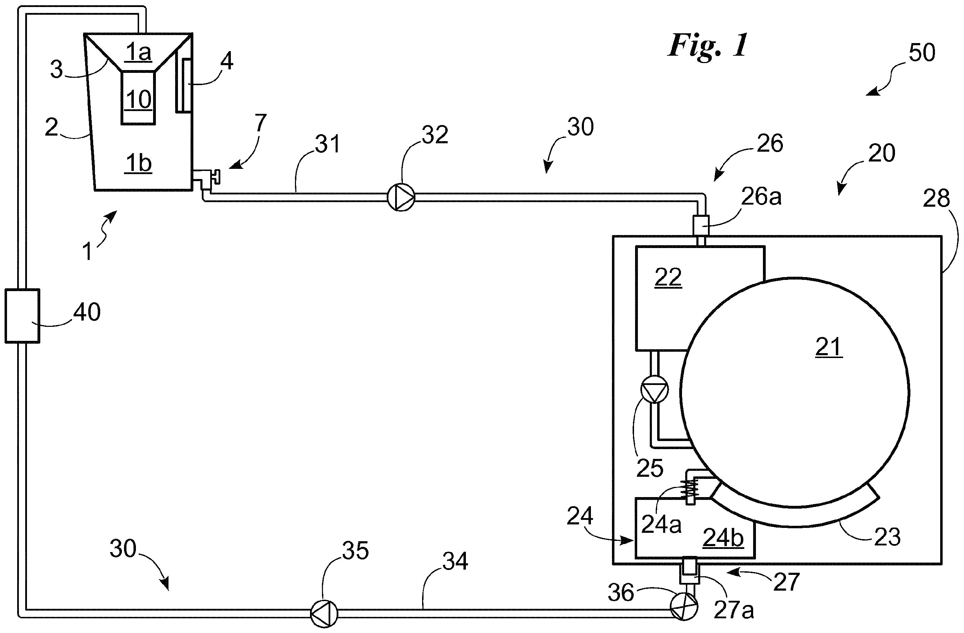

**Fig. 1** shows a schematic view of a variant of the autoclave for sterilisation according to the invention.

With reference to the Figures mentioned, the method according to the invention is implemented using a sterilising apparatus **50** described below initially in a structural manner.

In particular the sterilising apparatus 50 comprises an outer tank **1** and an autoclave for sterilisation or disinfection **20,** consisting of an autoclave suitable to sterilise at high pressure or a steam disinfector operating at environmental pressure or in a vacuum. Such devices in the present application are simply called autoclaves for sterilisation 20 for convenience.

The autoclave for sterilisation 20 is preferably of the known type.

It comprises, in short, a sterilisation chamber **21,** an inner tank **22** for water inside the autoclave 20, to be filled with distilled or demineralised water, heating means **23** and/or water pressurisation means, so as to create the vapour needed for sterilisation, means of disposal **24** of the high temperature steam, such as coolers **24a,** a storage tank **24b** and similar.

The sterilisation chamber 21 is in itself known and suitable to contain objects, in particular medical or medical-dental devices. It is also suitable to withstand high pressures and internal temperatures, especially if the sterilisation is performed at pressures between 1 relative bar and 2.2 relative bars. Instead the chamber 21 can be mechanically less performing if the sterilisation or disinfection takes place at substantially environmental pressure or in a vacuum, as happens in steam dis-infectors.

It is connected, in fluidic through connection to the inner tank 22 and is suitable to fill the same including through the heating means 23. The sterilisation chamber 21 is further connected, in fluidic through connection, to the steam disposal means 24.

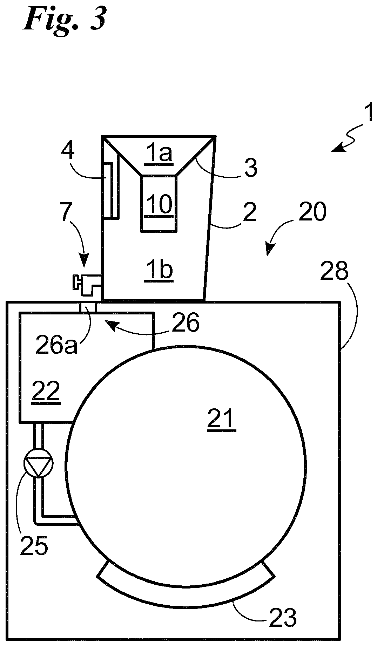

The autoclave for sterilisation 20 further comprises inlet means **26** to the inner tank 22 from outside, such as an inlet connector **26a,** in itself known, for connecting a pipe or similar or mere inlet by gravity (Fig. 3). The input connector 26a is, in turn, in fluidic through connection with the inner tank 22.

The autoclave for sterilisation 20 then comprises expulsion means **27** of the water.

Such expulsion means 27 appropriately comprise an outlet connector **27a,** also in itself known and in fluidic through connection with the sterilisation chamber 23 and/or disposal means 24.

The autoclave for sterilisation 20 also comprises circulation means **25** of the steam or water, such as a pump or is merely positioning according to the gravitational gradient to make the water circulate from the inlet means 26 to the inner tank 22 and then to the sterilisation chamber 21, disposal means 24 and expulsion means 27.

Among the various elements of the autoclave there are also of course electrically controlled valves and similar elements.

Said elements of the autoclave for sterilisation 20 are, furthermore, contained in a housing **28** usually of a parallelepiped shape.

The outer tank 1 is outside the autoclave 20 and thus physically separate from the same, even if, as described below, preferably connected to said autoclave 20 by pipes and the like.

It comprises a main housing **2,** preferably in polymer material and forming the outer structure of the tank 1 and a purification filter **10** as described further below.

The filter 10 is suitable to purify - and the term purify is taken to mean in the present application sterilise and /or demineralise the water by the mere passage of said water through filtering layers. In this application demineralisation is understood to mean a reduction of the bioburden with the aid of bacteriostatic means. The passage of the water is achieved by a pressure gradient between different portions of the outer tank 1 appropriately due simply to the gravitational force.

The filter 10 is thus placed in the tank 1 in an intermediate position in relation to the pressure gradient direction, in such a way that the tank 1 is able to contain in a high potential portion **1a** preferably placed in the upper part, non purified water, and in a low potential portion **1b**, preferably placed in the lower part, purified water passed through the filter 10. The two portions 1a and 1b are, with the exception of the filter 10 isolated from each other.

Preferably, the high-potential portion 1a is contained in an inner container 3 placed inside the main housing 2 and suitable to connect, in fluidic through connection, to the filter 10. The inner container 3 is preferably supported by resting on the main housing 2 and in turn comprises means of constraint, such as a circular wall threaded with the filter 10.

The tank 1 may in addition comprise sensors **4** of the quality of the filtered water preferably placed in the low potential portion 1b and consisting for example of conductivity and cloudiness sensors of the water and the like. Such sensors 4 are in addition preferably insertable and extractable from the tank 1 by means of simple, quick-fit attachments. In particular, a secondary container 5 is provided, suitable to contain the sensors 4, to support the main casing 2 and to place it in connection with the low-potential portion 1b. Said secondary container 5 is structurally appropriately in one piece with the inner container 3 even though the two contents are mutually isolated. The secondary container 5 may alternatively not be connected with the inside of the tank 1 and be simply a container for the sensors 4.

Stoppers **6** are then provided for the inner container, which in turn acts as a stopper to the main casing, and the secondary container 5.

The filter 10 is preferably connected to the tank by means of a quick-fit coupling and includes a plurality of filtering layers **11.**

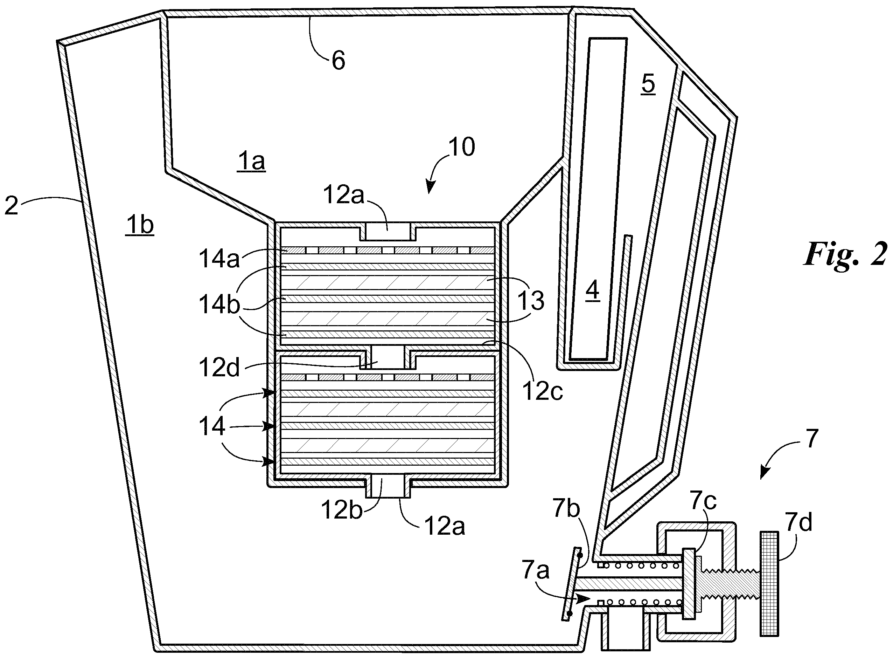

In structural detail, the filter 10 (fig. 2) is contained in a housing **12,** part of the filter 10, preferably cylindrical or prismatic. The housing 12 comprises an input **12a** and an output **12b** preferably having a smaller area than the total area of the base of the cylinder constituting the filter 10. The input and the output 12a and 12b are preferably circular and positioned in the centre of the upper and lower bases of the cylinder constituting the filter 10.

The input and the output 12a and 12b constitute an obligatory passage for the water, which passes, appropriately by gravity, from the high potential portion 1a to the low potential portion 1b.

The housing 12 may in addition be sub-divided into a plurality of reciprocally separable sections. In such case the housing 12 comprises intermediate bases **12c** reciprocally connected by known connection means, such as threads or the like and further comprises intermediate apertures **12d** of the type similar to the input and output 12a and 12b, suitable to allow the passage of water between the portions 1a and 1b. Such intermediate apertures12d further allow a slowing of the flow of water and easy maintenance of the filter 10 or the possibility of inserting filtering layers 11 of a smaller diameter, given that said intermediate apertures 12d define conical portions of a lesser diameter than the diameter of the housing 12 (Fig. 2).

The filtering layers 11 are suitable for entirely covering a normal cross-section in relation to the main axis of the cylindrical housing 12. In addition, given that in use the main axis of the housing 12 is preferably parallel to the direction of the gradient of potential between the portions 1a and 1b and thus preferably vertical, the filtering layers are positioned on the horizontal plane.

In functional detail, the filtering layers 11 divide mainly into two types: the active layers **13** and distribution layers **14** of the water.

The active layers 13 are each suitable to perform a purification or demineralisation function of the water. A variable number of active layers 13 may be provided for, preferably between seven and thirteen.

In particular, at least one active carbon layer 13 is provided for, and preferably a quantity between four and eight of active carbon layers and at least one active carbon layer impregnated with silver. Each of these preferably has a thickness of 1 mm to several centimetres. The main function of such layers is to eliminate chlorine and chloromethanes, to eliminate microorganisms and prevent their growth, to remove iodine and other substances. In addition the active carbon layers 13 are preferably placed both in the upper part of the filter 10 and in the lower part of said filter 10. In fact they are suitable to eliminate most of the impurities initially present in the water and to eliminate the possible particles and impurities formed by further active layers. Such layers may also be vegetable carbon, catalytic carbon or vegetable catalytic carbon.

The active layers 13 further comprise redox compounds. These remove the inorganic contaminants from the water and neutralise the pH. From one to three layers of such type are present, these too preferably in both the lower part and the upper part. The redox compounds substantially make available positive electrons or charges. On account of this exchange of electrons, many contaminants are converted into innocuous components which do not require further treatment. Other types of contaminants are removed from the flow and fixed to the active layer 13.

Other active layers 13 may be composed of iodine particles, possibly impregnated in resins, suitable for eliminating possible micro-organisms. Such particles preferably have an uneven charge number. Preferably solely one active layer 13 of this type is provided for.

Another type of active layer 13 preferably present is a layer in anionic resin, suitable to eliminate iodine and iodides from the water, and thus preferably placed below the layers with particles of iodine.

Yet another type of active layer 13 preferably present is a layer in resin for ion exchange, consisting of a mixture of anionic and cationic resins and suitable to remove inorganic and radiological contaminants. Such active layer 13 is preferably placed near the bottom of the filter 10.

The distribution layers 14 are instead suitable for slowing down or improving the distribution along the area of the normal cross-section of the cylinder.

In particular, a first type of distribution layer 14 is composed of a perforated disc **14a,** that is to say a polymer disc including a plurality of holes along the entire area. It is placed at the top of the filter 10, a short distance from the input 12a or at the intermediate apertures 12d. Such layer 13 is suitable to better distribute the water in input and output.

A second type of distribution layer 14 is composed of a layer of paper **14b.**

The paper layer 14b is much thinner than the other layers. It permits an optimal distribution of the flow of water along the area of the normal cross-section of the housing 12, and thus the optimisation of performance of the active filters 13. The paper layer 14b further retains the bigger impurities thus also partially acting as an active filter. Any type of paper can be used such as felt paper, nylon filter paper or otherwise. Preferably two paper layers 14 enclose, preferably not in direct contact, each active layer 13, except for the last active filter at the bottom of the filter 30, which obviously does not need a redistribution underneath.

The active layers 13 and the distribution layers 14 are thus substantially alternated along the length of the filter 10, except at the top, where the perforated disc 14a and a paper layer 14b are consecutive.

A similar filter 10 is described in the following patent applications: US-A-2008/0302714, in particular in Figs. 4 and 7 and from paragraph [0039] to paragraph [0108], and also in the patent applications US-A-5635063, US-A-6572769, US-A-7276161, US-A-7413663 of the company Zero Technologies, Inc.

The outer tank 1 lastly comprises an outlet **7** from the low-potential portion 1a on the outside, consisting of an outlet opening **7a,** preferably placed on the bottom of the low-potential portion 1a, of a valve **7b,** suitable to open or close the connection and of first control means **7c,** preferably manual and mechanical and suitable to control the valve 7b.

A similar outer tank 1 is described in the US patent application US-A-2012/0048787 by the company Zero Technologies, Inc. Said patent application describes a carafe that filters water to make it drinkable and/or microbiologically suitable before drinking. In the case of using said outer tank 1 described in the patent application US-A-2012/0048787 directly, it is appropriate to apply additional control means **7d,** suitable to convert the pressure control means 7a, present in said tank, into rotational or other control means suitable to keep the valve 7b open without continuous intervention. Said additional control means may consist of a threaded screw and a control knob suitable to make said screw advance and to keep the push-button constituting said first control means 7a pressed. Such a solution is illustrated in Fig. 2 schematically and functionally for the person skilled in the art.

The outer tank 1 and the autoclave 20 are preferably reciprocally connected in a fluidic through connection by connection means **30,** preferably composed of a series of tubes or the like and part of the apparatus 50.

Said connection means 30 comprise a first connection **31,** which connects the outer tank 1, and in particular the outlet 7, and thus the low-potential portion 1b, to the inlet means 26, and more particularly to the inlet connector 26a, and thus to the autoclave 20. Along the first connection, first transfer means **32** of the fluid may be provided from the outer tank 20 to the autoclave 1, preferably consisting of a pump or of a suitable arrangement of the elements taking advantage of the gravitational gradient for said transfer. The first connection 31 may further comprise a second valve (not illustrated), able to stop or restore upon command the first connection 40, otherwise replaced by the valve 7b of the outer tank 1.

Said first connection 31 thus places in fluidic through communication the outer tank 1 with the inner tank 22 and thus with the sterilisation chamber 21.

Alternatively, as illustrated in Fig. 3, the outer tank 1 is in fluidic through connection with the autoclave 20 merely by gravity and the connector 26a is without dedicated pipes and the like.

The connection means 30 further comprise, preferably, a second connection **34,** in a variant even without the first connection 31, suitable to connect the outer tank 1, and in particular the high potential portion 1a with the expulsion means 27, and in particular with the outlet connector 27a, so that the used water returns to the tank 1 and to the filter 10 to be filtered. Along the second connection 34, second transfer means **35** of the fluid from the autoclave 20 to the tank 1 may further be provided, preferably consisting of a pump. The second connection 34 further comprises a third valve **36,** suitable to interrupt or restore upon command the second connection 31.

The second connection 34 then places in fluidic through connection the disposal means 24, and thus the sterilisation chamber 21 with the outer tank 1. Along the second connection 34, a second filter **40** may be present, for example of the bacteriological type, such as a porous ceramic based filter, a UV filter or otherwise, to sterilise the water or other.

The functioning of the sterilising apparatus 50, described above in structural terms, is as follows.

The water is introduced into the high potential portion 1a of the outer tank 1, preferably directly from a tap with running water or the like.

The water crosses the filter 10 with the various membranes preferably by gravity, and is thus purified, that is to say demineralised and purified.

The pure water falls inside the low potential portion 1b of the outer tank 1. It comes out of the outer tank 1, when necessary, through the outlet 7.

The water passes through the outlet 7, the connection means 30, and in particular the first connection 31, and is conveyed by the first transfer means 32 to the inlet means 26 and in particular to the inlet connector 26a thus entering the autoclave 20.

The water then enters the inner tank and 22 here can be used easily, being purified water from the autoclave 20 of the known type. In particular, the water in the sterilisation chamber 21 is brought to high pressures and temperatures by the heating means 23.

Once the sterilisation is complete the sterilisation chamber 21 discharges the liquid or steam to the disposal means 24 which preferably send it to the expulsion means 27.

From here the liquid reaches the connection means 30 and in particular the second connection 34 and, by means of the second transfer means 35 and through the third valve 36 to the second filter 40, back to the outer tank 1 and in particular to the high-potential portion 1a. Alternatively, the liquid is disposed of after the transit to the second filter 40 avoiding potential risks to the environment.

Here the liquid is filtered once again and substantially reused at 100% except for possible losses which can quickly be replenished.

Should the sensors 4 signal that the pure water has a cloudiness or conductivity above the predefined parameters the machine signals that cleaning or rapid replacement of the filter is needed.

Said operation defines an innovative procedure obtained by means of the sterilisation apparatus 50 described and in particular by means of the outer tank 1 described.

Such method provides for a filtration step in the outer tank 1 described, outside the autoclave 20 and thus easy to install on conventional autoclaves already present and in use.

The invention also achieves a new application of the outer tank 1, described above, wherein said tank 1 is connected, in fluidic through connection, preferably through the connection means 30 described, to an autoclave 20, preferably of the type described, and is used to purify water and introduce it after said purification into the autoclave 20 for its use, preferably direct, i.e. without additional filtration, for sterilisation and thus in place of demineralised water. Said new application also comprises the connection of the tank 1 to the expulsion means 27 of the exhausted water of the autoclave 20 for its purification in the tank and recycling.

In addition, the disposal of the water through the second filter 40, even if not recycled, is not harmful to the environment.

The sterilising apparatus 50, the new method and the new application described achieve important benefits.

In particular, the water is supplied directly from running water and seldom, given that the autoclave 1 permits continuous recirculation. This latter advantage further permits a reduced environmental impact of the invention and drastically reduces the costs of demineralised water.

## Claims

1. Sterilising apparatus (50) comprising:
- an autoclave for sterilisation or disinfection (20), in particular for medical-dental use comprising a sterilisation chamber (21), an inner tank (22) for water, inside said autoclave for sterilisation (20) and heating means (23) of water,
- an outer tank (1) external to said autoclave for sterilisation (20), said outer tank (1) comprises a purification filter (10) suitable to purify water contained therein by mere passage of said water through filtering layers,
- wherein said outer tank (1) is divided into a high potential portion (1 a) and a low potential portion (1 b), said portions being in mutual fluidic through connection through said filter (10),
- wherein said purification filter (10) comprises a plurality of filter layers (11) comprising distribution layers (14) suitable to slow down and better distribute the water along the entire area of said filtering layers (11) and active layers (13) suitable to perform a purifying function of said water,
- said sterilising apparatus (50) comprises connection means (30) between said outer tank (1) and said autoclave for sterilisation (20),
- wherein said autoclave for sterilisation (20) comprises expulsion means (27) of the exhausted water from said autoclave for sterilisation (20), and means of connecting said outer tank (1) to said expulsion means (27) for the purification of the exhausted water in said outer tank (1) and recycling.

2. Sterilising apparatus (50) according to claim 1, wherein said distribution layers (14) and active layers (13) are alternating.

3. Use of a sterilising apparatus according to claims 1 or 2, to purify water contained in the outer tank (1) with the purification filter (10) by mere passage of said water through filtering layers and introduce the water, after purification, in the autoclave for sterilisation (20) in said inner tank (22) for its use for sterilisation, and to purify and recycle the exhausted water from the autoclave for sterilisation (20).

4. Use of a sterilising apparatus according to claim 3 to slow down and better distribute water along the entire area of the filter layers (11) with the distribution layers (14) of the purification filter (10) and to perform purifying functions of said water with the active layers (13).

## Patentansprüche

1. Sterilisierungsgerät (50) zur medizinischen und zahnärztlichen Verwendung, umfassend:
- einen Autoklav zur Sterilisierung oder Desinfizierung (20) von medizinischzahnärztlichen Vorrichtungen, umfassend eine Sterilisierkammer (21), einen Innenbehälter (22) für Wasser im Inneren des genannten Autoklavs zur Sterilisierung (20) und Elemente zum Erwärmen (23) von Wasser,
- einen Außenbehälter (1), außerhalb des genannten Gehäuses (28), der einen Reinigungsfilter (10) umfasst, der geeignet ist, das enthaltene Wasser durch einfachen Durchfluss des Wassers über Filterschichten zu reinigen,
- in dem der genannte Behälter (1) in einen Abschnitt mit hohem Potential (1a) und in einen Abschnitt mit niedrigem Potential (1b) unterteilt ist, wobei die genannten Abschnitte strömungstechnisch miteinander über den genannten Filter (10) verbunden sind,
- in dem der genannte Reinigungsfilter (10) eine Vielzahl von Filterschichten (11) umfasst, die Verteilungsschichten (14) einschließen, die geeignet sind, das Wasser entlang des gesamten Bereichs der genannten Filterschichten (11) und aktiver Schichten (13), die geeignet sind, Funktionen zum Klären des Wassers auszuüben, zu verlangsamen und besser zu verteilen,
- wobei das genannte Sterilisierungsgerät (50) Verbindungselemente (30) zwischen dem genannten Außenbehälter (1) und dem genannten Autoklav zur Sterilisierung (20) umfasst,
- in dem der genannte Autoklav zur Sterilisierung (20) Elemente (27) zum Ableiten des Altwassers aus dem genannten Autoklav zur Sterilisierung (20) und Elemente zum Anschließen des genannten Außenbehälters (1) an die genannten Elemente (27) zum Ableiten umfasst, um das genannte Altwasser in dem genannten Außenbehälter (1) zu reinigen und aufzubereiten.

2. Sterilisierungsgerät (50) nach Anspruch 1, bei dem der genannte Reinigungsfilter (10) Verteilungsschichten (14) und sich im Wesentlichen abwechselnde aktive Schichten (13) umfasst.

3. Verwendung eines Sterilisierungsgeräts (50) nach Anspruch 1 oder 2 zum Reinigen von in dem Außenbehälter (1) enthaltenem Wasser mit dem Reinigungsfilter (10) durch einfachen Durchfluss des Wassers über Filterschichten und Einleiten des genannten Wassers nach der genannten Reinigung in den genannten Autoklav zur Sterilisierung (20) in dem genannten Innenbehälter (22) für seine Verwendung zur Sterilisierung und um das Altwasser aus dem Autoklav zur Sterilisierung (20) zu reinigen und aufzubereiten.

4. Verwendung eines Außenbehälters (1) nach Anspruch 3, um das Wasser entlang des gesamten Bereichs der genannten Filterschichten (11) mit Verteilungsschichten (14) des genannten Reinigungsfilters (10) zu verlangsamen und besser zu verteilen, um Funktionen der Klärung des genannten Wassers über die aktiven Schichten (13) auszuüben.

## Revendications

1. Appareil de stérilisation (50) à usage médical-dentaire, comprenant :
- un autoclave pour la stérilisation ou la désinfection (20) de dispositifs médicaux-dentaires comprenant une chambre de stérilisation (21), un réservoir interne (22) pour l'eau, à l'intérieur dudit autoclave pour la stérilisation (20), et des moyens de chauffage (23) de l'eau,
- un réservoir externe (1), à l'extérieur dudit contenant (28) comprenant un filtre de purification (10) apte à purifier l'eau contenue par simple passage de l'eau à travers des couches filtrantes,
- dans lequel ledit réservoir (1) est divisé en une partie à haut potentiel (1a) et une partie à bas potentiel (1b), lesdites parties étant en connexion réciproque à passage fluide, à travers ledit filtre (10),
- dans lequel ledit filtre de purification (10) comprend une pluralité de couches filtrantes (11) incluant des couches de distribution (14) aptes à ralentir et mieux distribuer l'eau le long de l'entière surface desdites couches filtrantes (11) et des couches actives (13) aptes à exécuter des fonctions d'épuration de ladite eau,
- ledit appareil de stérilisation (50) comprend des moyens de raccordement (30) entre ledit réservoir externe (1) et ledit autoclave pour la stérilisation (20),
- dans lequel ledit autoclave pour la stérilisation (20) comprend des moyens d'éjection (27) d'eau usagée dudit autoclave pour la stérilisation (20), et des moyens pour raccorder ledit réservoir externe (1) auxdits moyens d'éjections (27) pour la purification de ladite eau usagée dans ledit réservoir externe (1) et le recyclage.

2. Appareil de stérilisation (50) selon la revendication 1, dans lequel ledit filtre de purification (10) comprend des couches de distribution (14) et des couches actives (13) essentiellement alternées.

3. Utilisation d'un appareil de stérilisation (50) selon la revendication 1 ou 2, pour purifier l'eau contenue dans le réservoir externe (1) avec le filtre de purification (10) par simple passage de l'eau à travers des couches filtrantes et pour injecter ladite eau, après ladite purification, dans ledit autoclave pour la stérilisation (20) dans ledit réservoir interne (22) pour son utilisation pour la stérilisation et pour purifier et recycler l'eau usagée de l'autoclave pour la stérilisation (20).

4. Utilisation d'un réservoir externe (1) selon la revendication 3 pour ralentir et mieux distribuer l'eau le long de la surface entière desdites couches filtrantes (11) avec des couches de distribution (14) dudit filtre de purification (10) et pour effectuer des fonctions d'épuration de ladite eau avec les couches actives (13).
